Europäisches Patentamt

European Patent Office

Office européen des brevets

(19)

(11)  **EP 1 574 167 A1**

(12)  # EUROPEAN PATENT APPLICATION

(43)  Date of publication:
  **14.09.2005  Bulletin 2005/37**

(51)  Int Cl.⁷: **A61B 10/00**

(21)  Application number: **05251483.3**

(22)  Date of filing: **11.03.2005**

(84)  Designated Contracting States:
  **AT BE BG CH CY CZ DE DK EE ES FI FR GB GR
  HU IE IS IT LI LT LU MC NL PL PT RO SE SI SK TR**
  Designated Extension States:
  **AL BA HR LV MK YU**

(30)  Priority: **12.03.2004  US 800339**

(71)  Applicant: **ETHICON ENDO-SURGERY, INC.
  Cincinnati, Ohio 45242 (US)**

(72)  Inventor: **Voegele, James W.
  Cincinnati Ohio 45249 (US)**

(74)  Representative: **Mercer, Christopher Paul et al
  Carpmaels & Ransford,
  43-45 Bloomsbury Square
  London WC1A 2RA (GB)**

(54)  **Electrode sleeve for biopsy device**

(57)  A handheld biopsy device is provided for the collection of soft tissue samples from a surgical patient. At least one electrode is provided, such as between a handle and the knife tip of the biopsy device. The electrodes can be provided on a sleeve. The sleeve with electrodes can be releasably supported on the biopsy device.

FIG. 1

## Description

### Field of the Invention

The present invention is related generally to devices and methods for the collection of soft tissue. More particularly, it relates to a device and apparatus to improve coagulation of tissue with biopsy devices used for the collection of soft tissue.

### Background of the Invention

The diagnosis and treatment of patients with cancerous tumors, pre-malignant conditions, and other disorders has long been an area of intense investigation. Non-invasive methods for examining tissue include palpation, X-ray, MRI, CT, and ultrasound imaging. When the physician suspects that a tissue may contain cancerous cells, a biopsy may be done using either an open procedure or a percutaneous procedure. For an open procedure, a scalpel is used by the surgeon to create a large incision in the tissue in order to provide direct viewing and access to the tissue mass of interest. The entire mass (excisional biopsy) or a part of the mass (incisional biopsy) may then be removed. For a percutaneous biopsy, a needle-like instrument is used through a very small incision to access the tissue mass of interest and to obtain a tissue sample for later examination and analysis. The advantages of the percutaneous method as compared to the open method may be significant and may include: less recovery time for the patient, less pain, less surgical time, lower cost, and less disfigurement of the patient's anatomy. Use of the percutaneous method in combination with imaging devices such as X-ray and ultrasound has resulted in highly reliable diagnoses and treatments.

Generally there are two ways to percutaneously obtain a portion of tissue from within the body, by aspiration or by core sampling. Aspiration of the tissue through a fine needle requires the tissue to be fragmented into pieces small enough to be withdrawn in a fluid medium. The method is less intrusive than other known sampling techniques but one can only examine cells in the liquid (cytology) and not the cells and the structure (pathology). In core biopsy, a core or fragment of tissue is obtained for histologic examination which may be done via a frozen or paraffin section.

The type of biopsy used depends mainly on various factors present in the patient, and no single procedure is ideal for all cases. Core biopsy, however, is very useful in a number of conditions and is widely used by physicians.

The biopsy device used should be lightweight, maneuverable, and handheld so that the surgeon may have the option to perform the biopsy procedure in combination with an ultrasound imaging device. In addition, the biopsy device should perform a biopsy procedure with fewer steps decreasing the overall time of the procedure.

The handheld biopsy device should be able to be held parallel to the chest wall of the patient, so that suspect tissue masses close to the chest wall can be easily sampled. It is desirable that the surgeon be able to easily steer the penetrating tip of the handheld device towards the desired tissue to be sampled. It is further desired that the surgeon have tactile feedback as the tissue is proved by the penetrating tip of the device, to provide the surgeon with clues regarding the disease state of the tissue encountered. The biopsy device should be "patient friendly" by not having noisy or jerky mechanical actuations during the procedure, and by not having to be used with large machines such as an X-ray stereotactic table.

Bleeding of tissue at the knife tip and around the piercer of the biopsy device may occur with current biopsy devices. Past devices have alleviated bleeding by placing an electrode through a cannula, removing the electrode, and then placing suction and cutting devices through the cannula to take the biopsy. Applicants have recognized a need for an instrument that cauterizes tissue and takes a biopsy core sample in a single step without removal and reinsertion of a device. To accomplish the single-insertion goal, applicants have further recognized a need for electrodes disposed on the outer surface of the piercer behind the tip. To enable removability of the electrodes, applicants have recognized the need for location of the electrodes on a sleeve that can be removably placed on the piercer. Because of the possibility of bleeding caused by the knife tip, applicants have recognized a need for the knife tip allowing for cauterization at the knife tip. Applicants have further recognized a need for a switching relay to alternately energize electrodes and knife tip to effectively cauterize different areas of the penetration site.

The following patent documents are incorporated herein by reference in their entirety: US 6,273,862 issued Aug 14, 2001; US 6,231,522 issued May 15, 2001; US 6,228,055 issued May 8, 2001; US 6,120,462 issued September 19, 2000; US 6,086,544 issued July 11, 2000; US 6,077,230 issued June 20, 2000; US 6,017,316 issued January 25, 2000; US 6,007,497 issued Dec. 28, 1999; US 5,980,469 issued Nov. 9, 1999; US 5,964,716 issued Oct 12, 1999; US 5,928,164 issued July 27, 1999; US 5,775,333 issued July 7, 1998; US 5,769,086 issued June 23, 1998; US 5,649,547 issued July 22, 1997; US 5,526,822 issued June 18, 1996; US 2003/0199785 published Oct 23, 2003; US 2003/0199754 published Oct 23, 2003; US 2003/0199754 published Oct 23, 2003 .

### Summary of the Invention

The present invention provides a biopsy device with electrodes to coagulate tissue and create hemostasis. The biopsy device may include a sleeve with electrodes attached. This sleeve slides over the piercer of the handheld biopsy device.

In one embodiment, the present invention provides a pair of electrodes attached to the exterior of a sleeve. The electrode sleeve can be used with the handheld biopsy device, such as a MAMMOTOME® biopsy instrument. The sleeve can be sized to slide over the piercer of the biopsy device and the sleeve can have a window sized to match the port on the piercer. The pair of electrodes can be disposed in association with the window, with one electrode extending along each side of the window. A gap between the electrodes allows treatment of tissue between the electrode gap with radio frequency (RF) energy to coagulate and create hemostasis. The knife tip of the handheld biopsy device can act as a third electrode, allowing treatment between each of the side electrodes and the knife tip structure. The sleeve uses an interface board to systematically control cautery by switching from one electrode to the other, or from one electrode to the knife tip.

The present invention also provides a biopsy device for the collection and retrieval of at least one soft tissue portion from a surgical patient, the biopsy device comprising:

    a. a handle;
    b. a piercer extending from the handle, the piercer having a distal tip for piercing tissue; and
    c. at least one electrode disposed intermediate the handle and the distal tip.

    Preferably the device comprises a plurality of electrodes disposed intermediate the handle and the distal tip.

    Preferably the distal tip is operatively connected to a source of electrical energy.

    Preferably the device comprises a first electrical connector for providing electrical energy to a first electrode disposed intermediate the handle and the distal tip.

    Preferably the device comprises a second electrical connector for providing electrical energy to a second electrode disposed intermediate the handle and the distal tip.

    Preferably the device comprises an electrical connector for providing electrical energy to the distal tip of the piercer.

    Preferably at least one electrode is supported on a sleeve disposed about the piercer.

    The present invention also provides apparatus for performing a medical procedure comprising:

    a hollow tissue piercing element having a sharpened distal end and a tissue receiving port spaced proximally of the distal end;
    a hollow sleeve having an open proximal end and at least one electrode;
        wherein the sleeve is positioned over the piercing element; and
    the sleeve and the piercing element are adapted to be positioned within a tissue mass.

The apparatus may comprise first, second and third electrodes.

Preferably the first, second, and third electrodes are on the sleeve, the apparatus further comprising:

    means for providing current to the first electrode and to the second electrode while the third electrode is uncharged;
    means for providing current to the first electrode and to the third electrode while the second electrode is uncharged; and
    means for providing current to the second electrode and to the third electrode while the first electrode is uncharged.

The apparatus may further comprise:

    a computer operatively attached to the electrodes; and
    the computer being adapted to sequence the charging of the electrodes.

Preferably at least one of said first, second, and third electrode comprises a piercing element.

## Brief Description of the Figures

FIGURE 1 is an isometric, expanded view of the probe assembly with the electrode sleeve shown displaced slightly distal of the piercer.
FIGURE 2 is an isometric view of the electrode sleeve.
FIGURE 3 is an isometric view of the probe assembly with the electrode sleeve attached.
FIGURE 4 is a schematic diagram of an interface relay board setup.
FIGURE 5 is an isometric view of a biopsy instrument including a hand piece for the collection of soft tissue.
FIGURE 6 is an isometric view of the probe assembly with the left handle shell removed to reveal internal components.
FIGURE 7 is an isometric view of the handpiece showing the probe assembly prior to attachment to a holster.
FIGURE 8 is an isometric view of an alternate embodiment of the electrode sleeve.
FIGURE 9 is an isometric view of an alternate embodiment of an exploded view of the probe assembly with the electrode sleeve slightly distal to the piercer.
FIGURE 10 is an isometric view of an alternate embodiment of the probe assembly with the electrode sleeve attached.
FIGURE 11 is an isometric view of an alternate embodiment of the probe assembly with the electrode sleeve slightly distal to handle 43.

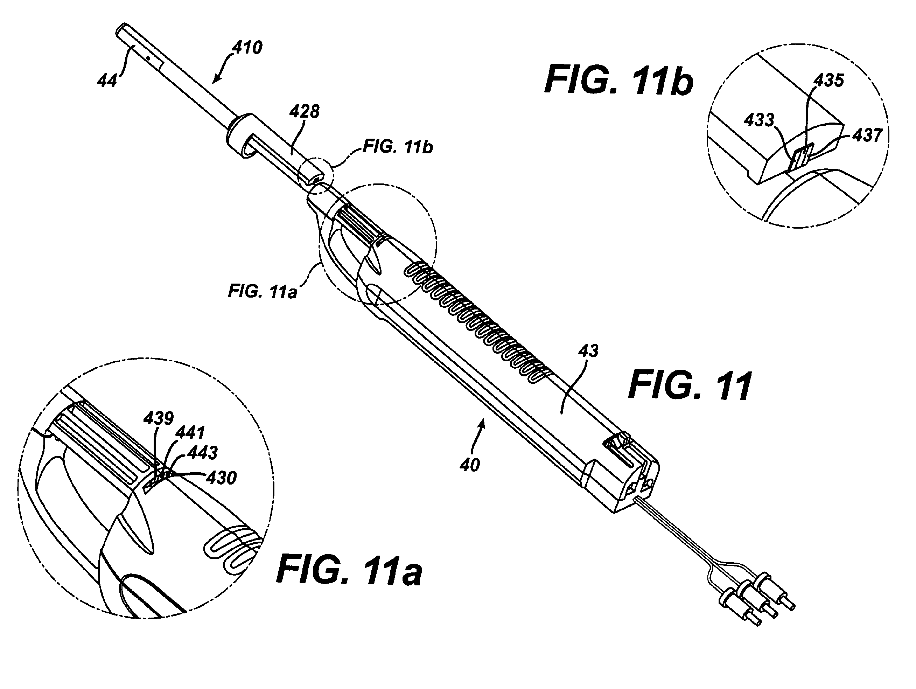

FIGURE 11 a is an isometric view of a breakout view of the contact in the groove on the underside of the handle.

FIGURE 11b is an isometric view of a breakout view of the contacts on the connector on the proximal end of the electrode sleeve.

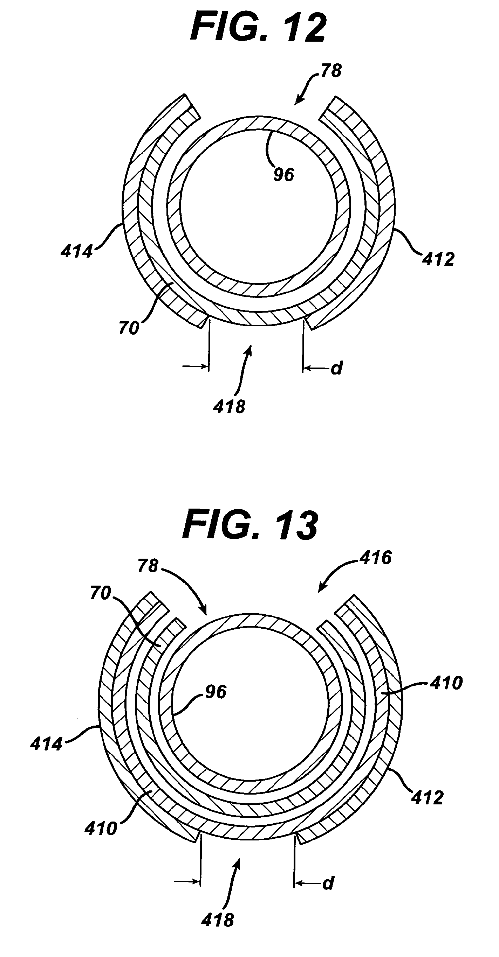

FIGURE 12 is a cross-sectional illustration of electrodes placed on a hollow piercer with a tissue cutter disposed for translation and rotation within the piercer.

FIGURE 13 is a cross-sectional illustration of electrodes placed on a hollow electrode sleeve disposed on a hollow piercer, with a tissue cutter disposed for translation and rotation within the piercer.

## Detailed Description of the Invention

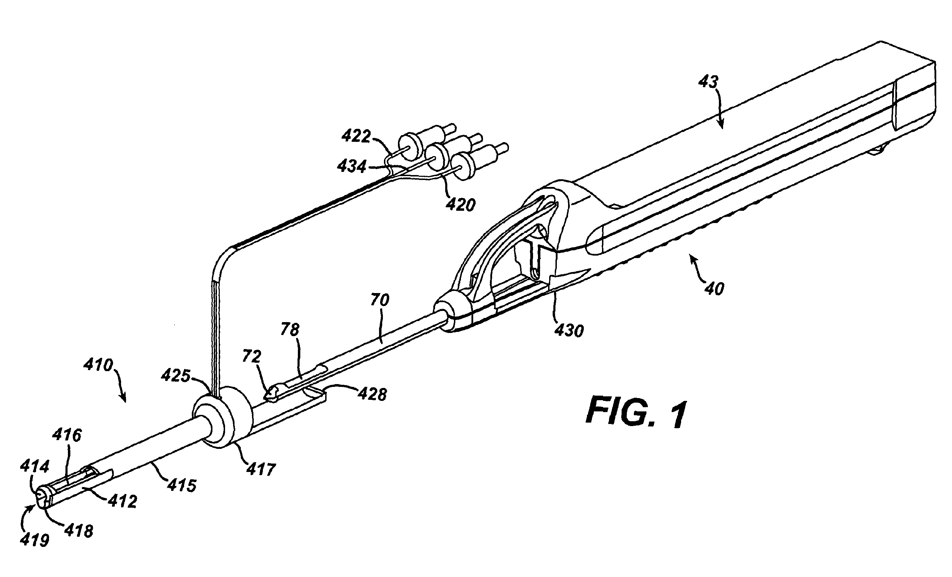

Referring now to the Figures, in which like numerals indicate like elements, Figure 1 discloses an exploded view of a probe assembly 40 and a sleeve 410. Probe assembly 40 may be a probe assembly as disclosed in U.S. Patent No. 6,273,862, "Surgical Device for the Collection of Soft Tissue," the entire contents of which are hereby incorporated herein by reference. A suitable probe assembly 40 is a part of the MAMMOTOME® breast biopsy instrument, available from Ethicon Endo-Surgery, Cincinnati, OH. Probe assembly 40 can include a hollow cannular piercer 70 extending distally from a hollow handle 43, a distal piercing knife tip 72 at the distal end of piercer 70, and a groove 430 on the underside of handle 43. Sleeve 410 can include a first electrode 412, a second electrode 414, a tissue receiving window 416, an electrode gap 418, a connector 428, a first wire 420 attached to electrode 412, a second wire 422 attached to electrode 414, and a third wire 434. Sleeve 410 is sized to slide over piercer 70. Sleeve 410 is placed over piercer 70 by sliding sleeve 410 proximally towards handle 43. The sleeve 410 can include a connector 428 for releasably connecting the sleeve 410 to the probe assembly 40. Connector 428 extends proximally from sleeve 40 and joins with groove 430 on the underside of handle 43 to connect sleeve 410 to handle 43. When sleeve 410 is connected to probe assembly 40, sleeve 410 abuts the distal end of handle 43, and knife tip 72 can protrude from an open distal end of sleeve 410 when sleeve 410 is connected to handle 43.

Electrode geometry may be as disclosed in World Patent Application No. 02/078557 to Gary Long filed on 29 March 2002, and incorporated herein by reference. The size, shape, and relative position of electrodes 412 and 414 are established by Ablation Index, I, and:

I=P/d

Where:

P is the perimeter of electrodes 412 and 414 and

d is the separation between adjacent edges of electrodes 412 and 414 on the bottom of the sleeve 410, the separation d corresponding to electrode gap 418 in the Figures.

In the embodiment of the invention having generally rectangular electrodes:

$$I=2(w+L)/d$$

Where:

w is the width of electrodes 412 and 414 and

L is the length of electrodes 412 and 414 measured parallel to the long axis of the sleeve 410..

Suitable ablation indices can be provided wherein: the separation d can be between about 1mm and about 3 mm: L can be between about 20 mm and about 40 mm: and w can be between about 3mm and 8 mm. In particular, d can be less than or equal to about 3 mm. More particularly, electrode size and spacing of d equal to 2mm, L equal to 30 mm, and w equal to 5 mm can be used to provide an Ablation Index I=35. In another specific embodiment, electrode size and spacing of d equal to 3mm, L equal to 30.4 mm, and w equal to 5.08 mm can be used to provide an Ablation Index I= 23.

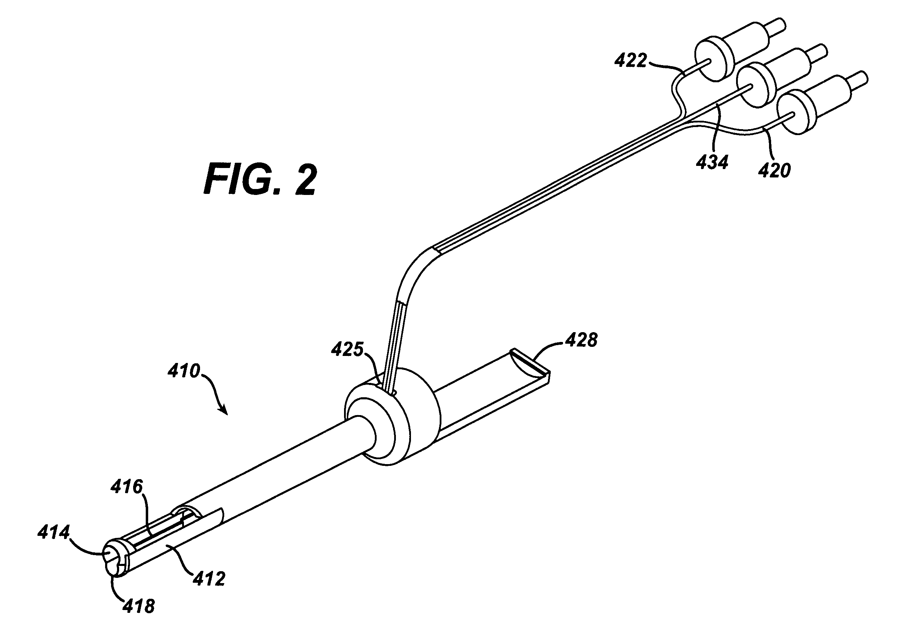

Figure 2 depicts features of electrode sleeve 410. Electrode sleeve 410 has dimensions allowing electrode sleeve 410 to slide proximally over piercer 70 towards handle 43. Cutter 96 can have dimensions of an 8 gauge (.165 inches), 11 gauge (.120 inches), or a 14 gauge (.083 inches) needle and the length of piercer 70 can be approximately 3.27 inches.

Sleeve 410 can include an elongated, hollow body portion 415 extending distally from a shoulder portion 417. Body portion 415 can include a generally rectangular window 416 and an end opening 419 at the distal end of body portion 415. Window 416 can be spaced proximally from the open distal end opening 419, near the distal end opening 419. Window 416 provides an opening in sleeve 410 which can be aligned with the tissue receiving port 78 of piercer 70. Window 416 allows port 78 to receive tissue extracted from the surgical patient. It is desirable for window 416 to be aligned with port 78 when performing the biopsy. Electrodes 412 and 414 can be positioned alongside of window 416 on the exterior surface sleeve 410, with electrode gap 418 separating electrodes 412 and 414. Electrode gap 418 corresponds to the separation d between electrode 412 and electrode 414, and electrode gap 418 corresponds to the spacing between electrodes 412 and 414 opposite window 416.

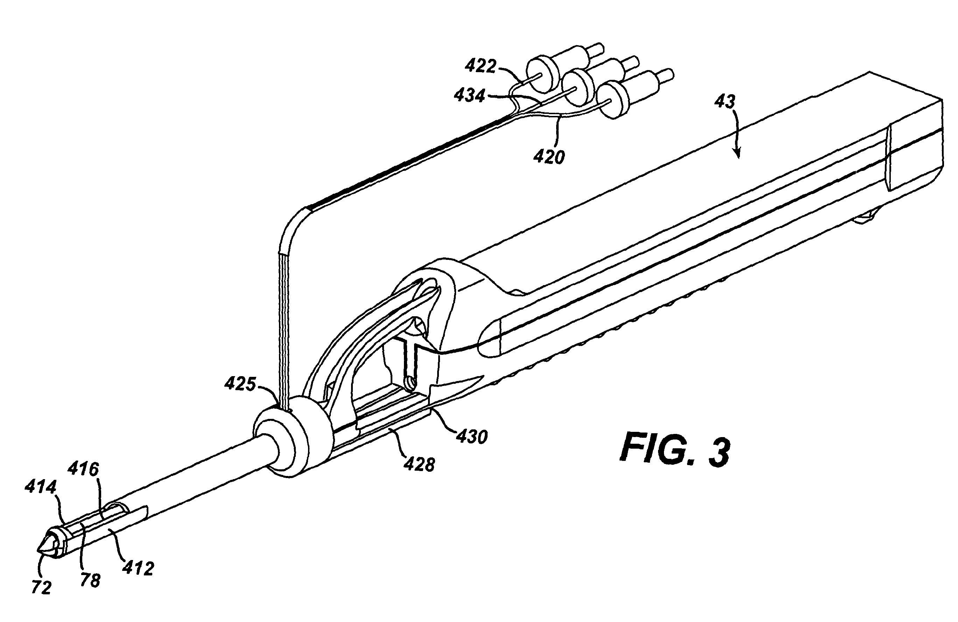

Figure 3 depicts electrode sleeve 410 attached to probe assembly 40. Connector 428 is joined with groove 430 on the underside of probe assembly 40.

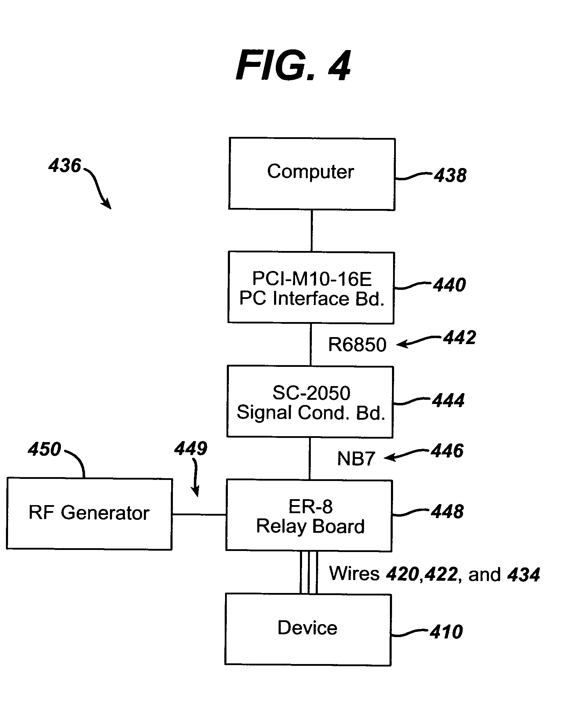

Wires 420 and 422 electrically connect to electrodes 412 and 414, respectively. A third wire 434 can be provided. The distal portion of wire 434 can be located in the interior of electrode sleeve 410, and the plastic covering or other insulation can be removed from the distal portion of wire 434 so that wire 434 can be in electrical contact with piercer 70 and/or the knife 72 of piercer 70, while the proximal portion of wire 434 extending proximally from electrode sleeve 410 can comprise a plastic covering or other insulating covering. Wires 434,420 and 422 can extend from electrode sleeve 410 at a wire opening 425. The proximal ends of wires 420, 422, and 434 can be electrically connected to an interface relay board as shown schematically in Figure 4.

Figure 4 schematically depicts in block diagram form components associated with operation of sleeve 410, the interconnected components indicated by reference number 436 in Figure 4. The components comprise a computer 438, a PC interface board 440, a cable 442, a Cable adaptor 444, a cable 446, a relay board 448, wires 420, 422, and 434, RF generator 450, a cable 449, and electrode sleeve 410. Computer 438 is connected to PC interface board 440. PC interface board 440 is connected to cable adaptor 444 by cable 442 Cable 446 connects cable adaptor 444 to relay board 448. RF generator 450 is connected to relay board 448 via a two-gauge cable 449. Wires 420, 422, and 434 connect to relay board 448 and to electrode sleeve 410.

PC interface board 440 is a multi-function component of assembly 436. For the specific embodiment disclosed, only the switching function of this multi-function component is used. PC interface board 440 switches electronic components on and off. Three of the eight switches contained in PC interface board 440 are used. Computer 438 can be programmed to control PC interface board 440 to indicate which switches of the three are on and which switches are off. Cable adaptor 444 is used to connect cable 442 and cable 446.

Relay board 448 acts as the physical relay from RF generator 450 to wires 420, 422, and 434. Relay board 448 uses switching instruction from PC interface board 440 to relay current and voltage from RF generator 450 to the correct electrode or electrodes.

RF generator 450 creates the radio frequency current used to provide RF energy to the electrodes. Relay board 448 directs RF energy to wires 420, 422, and 434, according to instructions provided by computer 438.

Boards and wires may be purchased as catalog components from National Instruments in Austin, Texas. Suitable part numbers are: for PC interface board 440/ part no. PCI-M10-16E; for cable 442/ part no. R6850; for cable adaptor 444/ part no. SC-2050; for cable 446/ part no. NB7; for Relay Board 448/ part no. ER-8. A suitable RF generator 450 is a Valley Lab Force 2 generator available from Valleylab located in Boulder, Colorado. Suitable software for providing control of RF energy to the electrodes is LabView Software v.6.0, available from National Instruments in Austin, Texas.

Once programmed and initiated, the components shown in Figure 4 can be used to provide a switching circuit to alternate charge to either electrodes 412 and 414, electrode 412 and piercer 70, or electrode 414 and piercer 70. Wires 420 and 422 supply current to electrodes 412 and 414 respectively, while wire 434 supplies current to tip 72 through piercer 70. When a metallic or otherwise conducting piercer 70 is employed, distal piercing knife tip 72 is energized when wire 434 is energized. Alternatively, wire 434 can be configured to make direct contact with a conductive tip 72.

In one embodiment, the components indicated by numeral 436 can be employed to alternately charge as a pair electrode 412 and electrode 414, then electrode 412 and knife tip 72, then electrode 414 and knife tip 72.

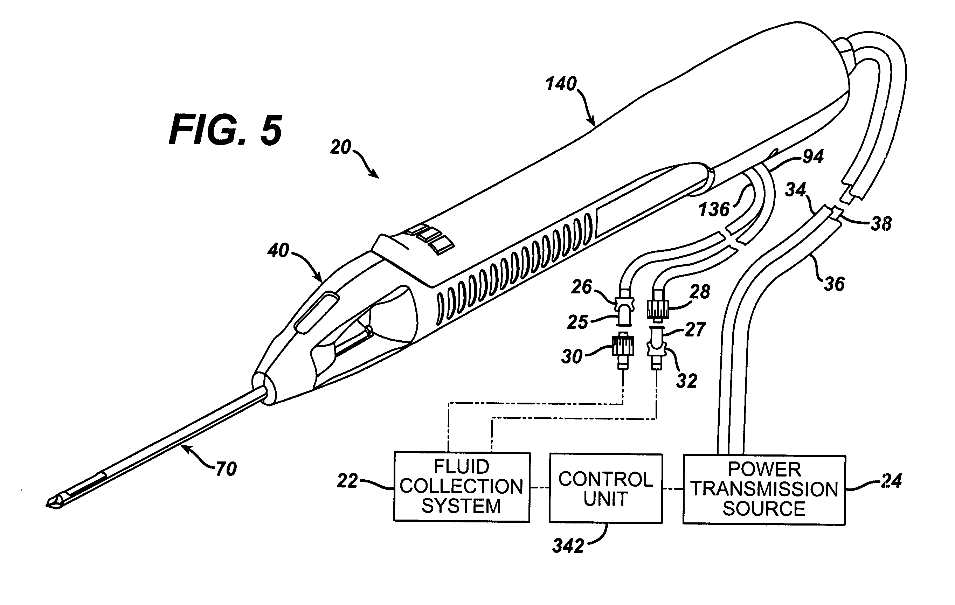

Figure 5 depicts an embodiment of a biopsy instrument comprising a probe assembly 40, a holster 140, a fluid collection system 22, a control unit 342, and a power transmission source 24 as disclosed in U.S. Patent No. 6,273,862, "Surgical Device for the Collection of Soft Tissue." The probe assembly 40 is detachably connected to the holster 140. Together they constitute a lightweight, ergonomically shaped, hand manipulatable portion referred to as a handpiece 20. The probe assembly 40 includes a piercer 70 extending distally from a hollow handle 43. The probe assembly 40 is fluidly connected to the fluid collection system 22 by a first vacuum tube 94 and a second vacuum tube 136. The first and second vacuum tubes are detachably connected to the fluid collection system 22 by a first connector 27 and a second connector 25, respectively. The first connector has a male portion 32 and a female portion 28 attached to the first vacuum tube 94. The second connector 25 has a female portion 30 and a male portion 26 attached to the second vacuum tube 136. The connector portions, 26, 28, 30, and 32, are attached in this manner to prevent the accidental switching of the first and second tubes, 136 and 94, to the fluid collection system 22. The holster 140 includes a first rotatable shaft 34, a second rotatable shaft 36, and a control cord 38. The first and second rotatable shafts, 34 and 36, are preferably flexible so that the operator may easily manipulate the handpiece 20 with one hand. The control cord 38 operatively connects the handpiece 20 to the power transmission source 24 and control unit 342.

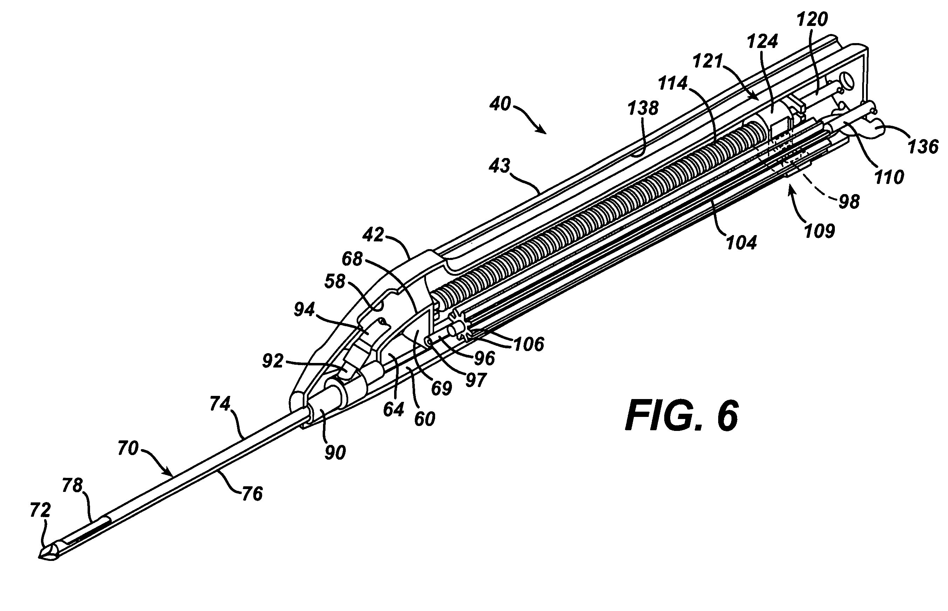

Referring to Figure 6, an isometric view of the probe assembly 40 with the left portion of handle shell 44 removed reveals the placement of the components. Part of the first vacuum tube 94 has also been removed for clarity. The carriage 124 is shown in the fully retracted position so that the cutter 96 is also at the fully retracted, or first position. The cutter blade 97 is slightly distal to the vertical wall 69 on the handle 43. The foot of the carriage 124 is adapted to slide along a carriage guide surface 60 on the inside bottom of the hollow handle 43. As shown, a cutter axial transmission 121 includes the carriage 124, the screw 114, and the screw shaft 120. A cutter rotational transmission 109 includes the drive gear 104, the cutter gear 98, and the gear shaft 110.

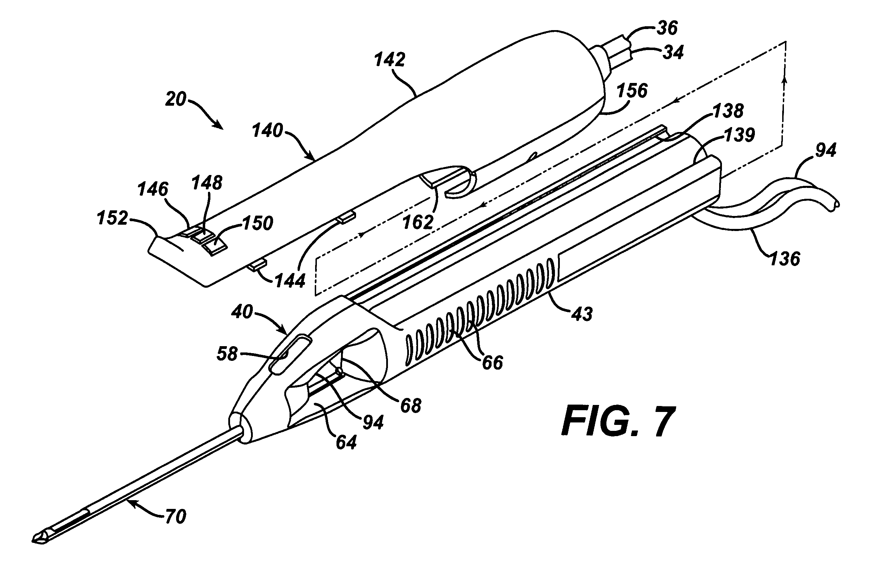

Referring to Figure 7, the holster 140 and the probe assembly 40 are shown separated. A pair of tabs 144 project laterally from each side of a holster upper shell 142, and insert into right and left undercut ledges, 138 and 139 respectively, of the hollow handle 43 of the probe assembly 40. A plurality of indentations 66 are provided on the handle 43 to improve the operator's grip on the instrument. A tube slot 162 in the lower shell 156 of the holster L40 provides clearance for first and second vacuum tubes, 94 and 136. A first switch 146, a second switch 148, and a third switch 150 are mounted in the distal portion of the holster 140 so that the physician can operate the handpiece 20 with a single hand while having the other hand free to operate an ultrasonic imaging device or the like. The switches 146, 148, and 150 are provided to operate the power transmission source 24 and the fluid collection system 22 in conjunction with the control unit 342. A ridge 152 on the distal end of the holster 140 is provided to assist the operator in grasping the handpiece 20 and in operating the switches 146, 148, and 150. The ridge 152 further provides the operator with a tactile reference as to where to properly grasp the handpiece 20.

Still referring to Figure 7, the probe assembly 40 includes a window 58 so that a portion of the first vacuum tube 94 may be viewed. The first and second vacuum tubes, 94 and 136, can be made from a flexible, transparent, or translucent material, such as silicone tubing. This enables visualization of the material flowing through the tubes. By having the window 58 in the probe assembly 40, the operator can see the flow in the first tube 94 without needing to look away from the tissue into which the piercer 70 is inserted. A transverse opening 68 is provided in the distal end of the hollow handle 43 which allows access from either side to a tissue sampling surface 64. The tissue extracted from the surgical patient is retrieved by the operator or an assistant from the tissue sampling surface.

Prior to obtaining a biopsy sample, the electrode sleeve 410 can positioned over piercer 70 with window 416 aligned with tissue receiving port 78, and wires 422, 420, and 434 can be connected as shown in Figure 4. Piercer 70 can be positioned in breast tissue to be biopsied. Vacuum can be provided at tissue port 78 so that soft tissue adjacent to port 78 prolapses into port 78 through window 416 when the first vacuum tube 94 is fluidly connected to the vacuum of the fluid collection system 22. The tissue pulled into port 78 is then severed by rotating and advancing cutter blade 97 and stored inside the cutter lumen of the cutter 96. Cutter 96 can then be retracted proximally to a first position so that cutter blade 97 is just distal to the tissue sampling surface 64, and a stationary tissue knockout pin can be used to push the severed tissue sample from the cutter onto the surface 64.

After one or more biopsy samples have been obtained, the operator can then coagulate the breast tissue at the sample site by energizing the electrodes 412 and 416 associated with the sleeve 410. If desired, tissue can be cauterized during insertion of the piercer 70 into tissue. For instance, the electrodes associated with sleeve 410 and/or the tip 72 can be energized during insertion of the piercer 70 to reduce bleeding at the insertion site.

In one embodiment, RF generator 450 can provide about 70 amps, while the switching generated from interface relay board setup 436 changes each electrode pair for a time of about 2500 milliseconds. The process of systematically switching charged electrodes is repeated until completion of coagulation. The surgeon can rotate the biopsy device with electrode sleeve 410 attached to align electrodes with different portions of the tissue to ensure hemostasis of the entire tissue area. Once the core biopsy sample has been retrieved and hemostasis exists in tissue, the biopsy device can be removed from the breast tissue or prepared for another core biopsy.

Figure 13 provides a cross-sectional view of the electrode sleeve 410 positioned on a piercer 70, with the cross-sectional view taken perpendicular to the axis of the piercer 70 and through the tissue port 78. Electrode sleeve 410 can be formed of a suitable non metallic material, such as a plastic or polymeric material. For instance, sleeve 410 can be formed of a liquid crystal polymer available as Vectra® brand liquid crystal polymer available from Ticona Company of Germany. In Figure 13, coagulation can be provided at gap 418 when electrodes 412 and 414 are energized. Alternatively, when current from the generator is provided to the piercer 70 and to one of the electrodes 412 or 414, coagulation can occur in tissue extending into sleeve window 416 and aligned tissue port 78 due to the conductive path through the tissue at either edge of the tissue port 78, depending on which electrode 412 or 414 is activated. Applicant has found that it is not necessary to rotate any of the components within the tissue mass (such as the piercer 70 and sleeve 410) to obtain substantially 360 degree coagulation around the piercer/sleeve. Without being limited by theory, it is believed that such 360 degree coagulation of tissue (such as breast tissue) without rotation of the piercer 70 and sleeve 410 can be accomplished because of the change of impedance through the tissue as coagulation progresses and heating of the electrodes 412 and 414. In an alternative embodiment, electrodes 412 and 414 could be attached directly to piercer 70, as shown in Figure 12. Piercer 70 could then be formed of a non-conductive material, such as an engineering plastic. For example, Vectra® brand liquid crystal polymer, available from Ticona in Germany could be used as the piercer material. Attaching electrodes 412 and 414 to piercer 70 eliminates the need for a separate sleeve with electrodes 412 and 414 attached. The third electrode could be formed by knife tip 72 being electrically connected to handle 43. Knife tip 72 would be charged when cutter 96 contacts the proximal surface of knife tip 72 to coagulate tissue postioned generally between the knife tip 72 and the electrodes 412/414. Further, the portion of the cutter within tissue port 78, when energized along with energization of one or both of the electrodes 412/414, can provide coagulation of tissue proximal of the knife tip 72. In this embodiment, wires 420, 422, and 434 can be connected as previously disclosed.

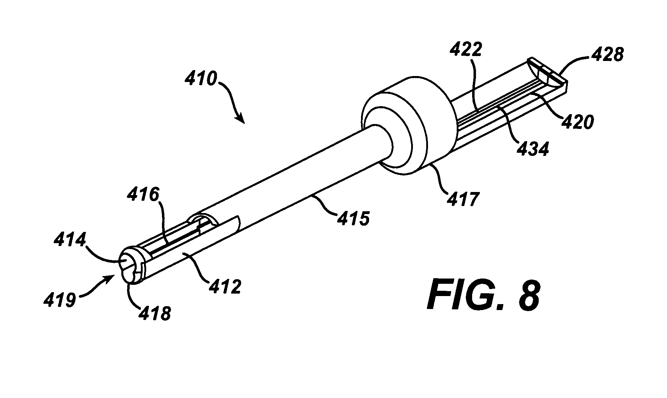

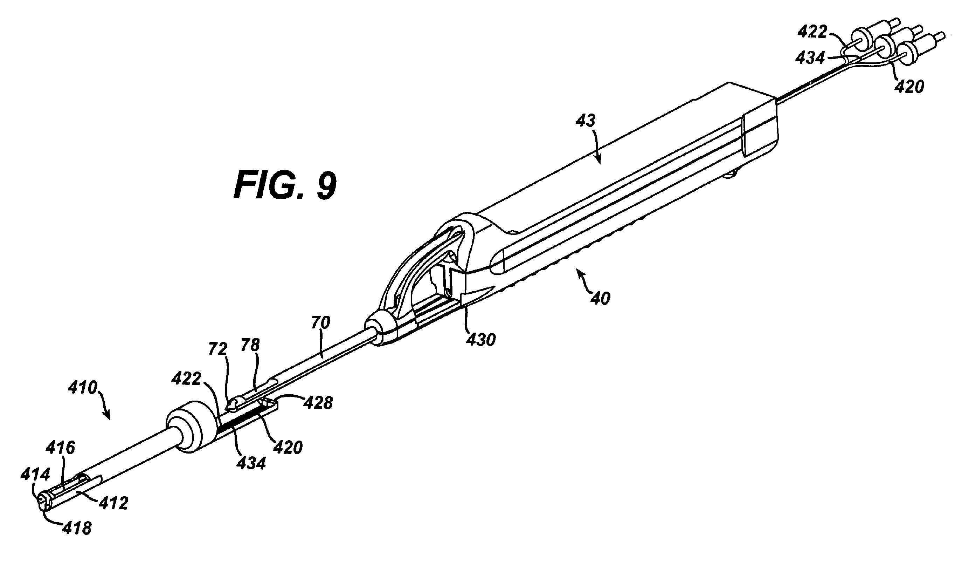

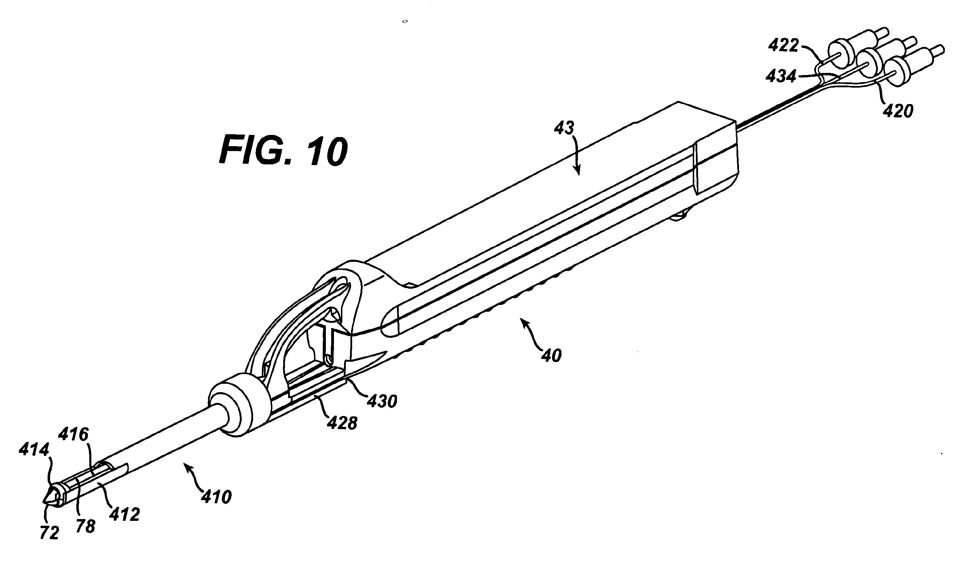

Another embodiment is shown in Figures 8, 9, and 10. In this embodiment, wires 420, 422, and 434 could extend through the handle 43 of the biopsy device and emerge through the proximal end of the biopsy device. Wires 420, 422, and 434 could be attached to connector 428 and fixed each to one of contacts 433, 435, and 437. Contacts 439, 441, and 443 in groove 430 on the underside of handle 43 would contact contacts 433, 435, and 437 on connector 428 when electrode sleeve 410 is connected to handle 43. Figure 11 shows the underside of probe assembly 40 and electrode sleeve 410 with electrode sleeve 410 slightly distal to handle 43. Connector 428 is shown with contacts 433, 435, and 437 schematically illustrated at a proximal end of sleeve 410.

Figure 11a shows groove 430 on the underside of handle 43, and contacts 439, 441, and 443 placed in groove 430. When electrode sleeve 410 abuts handle 43, connector 428 is in groove 430. Contacts 433,435, 437 are in electrical contact with contacts 439, 441, and 443, respectively, to provide for energizing the electrodes.

In another embodiment, the three or more electrodes can be positioned on the sleeve 410. With three or more electrodes, an interface relay circuit board can be used to switch charge among the electrodes in schemes that alternate coagulation to different portions of the tissue in contact in surface with the sleeve or the piercer.

In another embodiment, only one electrode could be utilized in a monopolar arrangement. A grounding pad, placed under the patient as is practiced while using monopolar RF energy, could be utilized while the single electrode is charged. Alternately, the single electrode could be utilized with the knife tip and a bipolar RF arrangement.

In another embodiment, any electrode on the piercer or on the sleeve may have a variety of geometries that efficiently coagulate the tissue. An electrode, for example, may surround at least a portion of the circumference of the sleeve or piercer as would a ring, and have an axial length along the sleeve or piercer.

In another embodiment, a switching cycle utilized by the components illustrated in Figure 4 by numeral 436 can be programmed so that the switching rate is zero, so that only two electrodes become charged. In this embodiment, a surgeon using the biopsy device may choose to charge two electrodes and may manually change the set of electrodes charged. The set of electrodes being charged can be changed through the program. The surgeon inputs to computer 438 the set of electrodes to be charged. Each time a new set of electrodes is selected, the new set can be input to computer 438.

It will also be recognized by one skilled in the art that some or all of the components identified by reference numeral 436 may be incorporated as an integral part of hardware and software used to control the cutting and suction portions of a process used with a biopsy device. A computer console may also be employed for controlling some or all aspects of cutting, suction, cauterization, and electrode switching.

While the present invention has been illustrated by description of several embodiments, it is not the intention of the applicant to restrict or limit the spirit and scope of the appended claims to such detail. Numerous other variations, changes, and substitutions will occur to those skilled in the art without departing from the scope of the invention. For instance, the device and method of the present invention has been illustrated in relation to coagulation of breast tissue, but it will be understood the present invention has applicability in other tissues as well. Moreover, the structure of each element associated with the present invention can be alternatively described as a means for providing the function performed by the element.

It will be understood that the foregoing description is provided by way of example, and that other modifications may occur to those skilled in the art without departing from the scope of the appended Claims.

**Claims**

1. A biopsy device comprising:

   a hollow sleeve adapted to receive a tissue piercing element therein, the sleeve comprising an open proximal end, a distal end, and a tissue receiving opening disposed intermediate the proximal end and the distal end; and

   at least one electrode disposed on the sleeve.

2. The biopsy device of Claim 1 wherein at the sleeve has an open distal end.

3. The biopsy device of Claim 1 wherein at least one electrode is associated with the tissue receiving opening.

4. The biopsy device of Claim 1 comprising at least two electrodes.

5. The biopsy device of Claim 1 comprising first and second electrodes associated with the edges of the tissue receiving opening.

6. The sleeve of Claim 1 further comprising a connector for releasably attaching the sleeve to a biopsy device.

7. The device of Claim 1 wherein the at least one electrode is operatively connected to a source of electrical energy.

8. A biopsy device for the collection and retrieval of at least one soft tissue portion from a surgical patient, the biopsy device comprising:

   d. a handle;
   e. a piercer extending from the handle, the piercer having a distal tip for piercing tissue; and
   f. at least one electrode disposed intermediate the handle and the distal tip.

9. The device of Claim 8 comprising a plurality of electrodes disposed intermediate the handle and the distal tip.

10. Apparatus for performing a medical procedure comprising:

   a hollow tissue piercing element having a sharpened distal end and a tissue receiving port spaced proximally of the distal end;
   a hollow sleeve having an open proximal end and at least one electrode;
   wherein the sleeve is positioned over the piercing element; and
   the sleeve and the piercing element are adapted to be positioned within a tissue mass.

*FIG. 1*

FIG. 2

EP 1 574 167 A1

*FIG. 3*

# FIG. 4

*436*

Computer — *438*

PCI-M10-16E
PC Interface Bd. — *440*

R6850 ← *442*

SC-2050
Signal Cond. Bd. — *444*

NB7 ← *446*

*450* *449*

RF Generator

ER-8
Relay Board — *448*

Wires *420,422*, and *434*

Device — *410*

**FIG. 5**

EP 1 574 167 A1

**FIG. 6**

FIG. 7

EP 1 574 167 A1

**FIG. 8**

**FIG. 9**

FIG. 10

FIG. 11b

FIG. 11

FIG. 11a

435
437
433

43
40
428
410
44

FIG. 11b

FIG. 11a

439
441
443
430

# FIG. 12

# FIG. 13

## EUROPEAN SEARCH REPORT

**European Patent Office**

Application Number

EP 05 25 1483

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 6 485 436 B1 (TRUCKAI CSABA ET AL) 26 November 2002 (2002-11-26) * column 2, line 56 - column 3, line 21 * * column 6, line 50 - line 63 * * figures * ----- | 1-10 | A61B10/00 |
| X | US 6 471 700 B1 (BURBANK FRED ET AL) 29 October 2002 (2002-10-29) * figures * ----- | 1-3,6-8, 10 | |
| X | US 2003/093007 A1 (WOOD BRADFORD J) 15 May 2003 (2003-05-15) * paragraph [0036] - paragraph [0038]; figures 1-3 * ----- | 8 | |
| A | US 2002/193705 A1 (BURBANK FRED ET AL) 19 December 2002 (2002-12-19) * the whole document * ----- | 1-10 | |
| A | WO 03/020136 A (REX MEDICAL, L.P) 13 March 2003 (2003-03-13) * the whole document * ----- | 1-10 | **TECHNICAL FIELDS SEARCHED (Int.Cl.7)** A61B |

The present search report has been drawn up for all claims

| Place of search | Date of completion of the search | Examiner |
|---|---|---|
| Munich | 20 July 2005 | Held, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 05 25 1483

This annex lists the patent family members relating to the patent documents cited in the above-mentioned European search report.
The members are as contained in the European Patent Office EDP file on
The European Patent Office is in no way liable for these particulars which are merely given for the purpose of information.

20-07-2005

| Patent document cited in search report | | Publication date | Patent family member(s) | | Publication date |
|---|---|---|---|---|---|
| US 6485436 | B1 | 26-11-2002 | NONE | | |
| US 6471700 | B1 | 29-10-2002 | US | 2001003791 A1 | 14-06-2001 |
| | | | US | 6427081 B1 | 30-07-2002 |
| | | | US | 6161034 A | 12-12-2000 |
| | | | US | 6261241 B1 | 17-07-2001 |
| | | | US | 6331166 B1 | 18-12-2001 |
| | | | AU | 2771601 A | 16-07-2001 |
| | | | CA | 2395225 A1 | 12-07-2001 |
| | | | EP | 1255499 A2 | 13-11-2002 |
| | | | JP | 2003518974 T | 17-06-2003 |
| | | | WO | 0149184 A2 | 12-07-2001 |
| | | | US | 2003144605 A1 | 31-07-2003 |
| | | | US | 2002115943 A1 | 22-08-2002 |
| | | | US | 2002120211 A1 | 29-08-2002 |
| | | | US | 2003023239 A1 | 30-01-2003 |
| | | | US | 6497706 B1 | 24-12-2002 |
| | | | US | 2001002250 A1 | 31-05-2001 |
| | | | US | 2004117652 A1 | 17-06-2004 |
| | | | US | 6540695 B1 | 01-04-2003 |
| | | | US | 2002058884 A1 | 16-05-2002 |
| | | | US | 2002068879 A1 | 06-06-2002 |
| | | | US | 2005038462 A1 | 17-02-2005 |
| | | | AT | 288228 T | 15-02-2005 |
| | | | AU | 5777500 A | 31-01-2001 |
| | | | CA | 2376146 A1 | 04-01-2001 |
| | | | DE | 60017904 D1 | 10-03-2005 |
| | | | EP | 1189546 A1 | 27-03-2002 |
| | | | EP | 1525856 A2 | 27-04-2005 |
| | | | JP | 2003503098 T | 28-01-2003 |
| | | | US | 2002038087 A1 | 28-03-2002 |
| | | | WO | 0100101 A1 | 04-01-2001 |
| | | | US | 2002188196 A1 | 12-12-2002 |
| | | | US | 2005063908 A1 | 24-03-2005 |
| | | | US | 2004101479 A1 | 27-05-2004 |
| | | | US | 6725083 B1 | 20-04-2004 |
| | | | US | 2004116806 A1 | 17-06-2004 |
| | | | US | 2004193044 A1 | 30-09-2004 |
| | | | US | 2005143656 A1 | 30-06-2005 |
| | | | US | 2002161298 A1 | 31-10-2002 |
| | | | AU | 3583500 A | 25-08-2000 |
| | | | CA | 2361530 A1 | 10-08-2000 |
| | | | EP | 1146910 A2 | 24-10-2001 |
| | | | JP | 2003517453 T | 27-05-2003 |
| | | | WO | 0045854 A2 | 10-08-2000 |
| | | | AU | 5926699 A | 10-04-2000 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.**

EP 05 25 1483

This annex lists the patent family members relating to the patent documents cited in the above-mentioned European search report.
The members are as contained in the European Patent Office EDP file on
The European Patent Office is in no way liable for these particulars which are merely given for the purpose of information.

20-07-2005

| Patent document cited in search report | | Publication date | Patent family member(s) | | Publication date |
|---|---|---|---|---|---|
| US 6471700 | B1 | | CA | 2344641 A1 | 30-03-2000 |
| | | | EP | 1115345 A2 | 18-07-2001 |
| | | | JP | 2002526191 T | 20-08-2002 |
| | | | WO | 0016697 A2 | 30-03-2000 |
| | | | US | 2001017137 A1 | 30-08-2001 |
| | | | US | 6517498 B1 | 11-02-2003 |
| US 2003093007 | A1 | 15-05-2003 | NONE | | |
| US 2002193705 | A1 | 19-12-2002 | US | 6454727 B1 | 24-09-2002 |
| | | | US | 6331166 B1 | 18-12-2001 |
| | | | AU | 1738200 A | 13-06-2000 |
| | | | CA | 2348482 A1 | 02-06-2000 |
| | | | EP | 1130997 A1 | 12-09-2001 |
| | | | JP | 2002530139 T | 17-09-2002 |
| | | | WO | 0030531 A1 | 02-06-2000 |
| | | | US | 2002120211 A1 | 29-08-2002 |
| | | | US | 6517498 B1 | 11-02-2003 |
| | | | US | 2002111564 A1 | 15-08-2002 |
| | | | AU | 2976599 A | 20-09-1999 |
| | | | CA | 2322804 A1 | 10-09-1999 |
| | | | EP | 1059881 A1 | 20-12-2000 |
| | | | JP | 2002505136 T | 19-02-2002 |
| | | | US | 2002087095 A1 | 04-07-2002 |
| | | | US | 2002052564 A1 | 02-05-2002 |
| | | | US | 2002058885 A1 | 16-05-2002 |
| | | | US | 2003144605 A1 | 31-07-2003 |
| | | | US | 2002115943 A1 | 22-08-2002 |
| | | | WO | 9944506 A1 | 10-09-1999 |
| | | | US | 2002007130 A1 | 17-01-2002 |
| | | | US | 6261241 B1 | 17-07-2001 |
| | | | US | 2003023239 A1 | 30-01-2003 |
| | | | US | 6344026 B1 | 05-02-2002 |
| | | | US | 2001017137 A1 | 30-08-2001 |
| | | | US | 2003176812 A1 | 18-09-2003 |
| | | | US | 6471700 B1 | 29-10-2002 |
| | | | US | 2005090762 A1 | 28-04-2005 |
| | | | US | 6497706 B1 | 24-12-2002 |
| | | | US | 2004153004 A1 | 05-08-2004 |
| | | | US | 2001002250 A1 | 31-05-2001 |
| | | | US | 6540695 B1 | 01-04-2003 |
| | | | US | 2004204709 A1 | 14-10-2004 |
| | | | US | 2004167431 A1 | 26-08-2004 |
| | | | US | 2004167432 A1 | 26-08-2004 |
| | | | US | 2004171967 A1 | 02-09-2004 |
| | | | US | 2001014779 A1 | 16-08-2001 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.**

EP 05 25 1483

This annex lists the patent family members relating to the patent documents cited in  the above-mentioned European search report.
The members are as contained in the European Patent Office EDP file on
The European Patent Office is in no way liable for these particulars which are merely  given for the purpose of information.

20-07-2005

| Patent document cited in search report | | Publication date | Patent family member(s) | | Publication date |
|---|---|---|---|---|---|
| US 2002193705 | A1 | | US | 2004215187 A1 | 28-10-2004 |
| | | | US | 2001039420 A1 | 08-11-2001 |
| | | | US | 2005004492 A1 | 06-01-2005 |
| | | | US | 2005010131 A1 | 13-01-2005 |
| | | | US | 2002068879 A1 | 06-06-2002 |
| | | | US | 2002072688 A1 | 13-06-2002 |
| | | | US | 2005038462 A1 | 17-02-2005 |
| | | | US | 2002068880 A1 | 06-06-2002 |
| WO 03020136 | A | 13-03-2003 | US | 2003045811 A1 | 06-03-2003 |
| | | | US | 2003045873 A1 | 06-03-2003 |
| | | | CA | 2455067 A1 | 13-03-2003 |
| | | | EP | 1420699 A1 | 26-05-2004 |
| | | | JP | 2005501593 T | 20-01-2005 |
| | | | WO | 03020136 A1 | 13-03-2003 |

EPO FORM P0459

For more details about this annex : see Official Journal of the European Patent Office, No. 12/82